(19) Europäisches Patentamt
European Patent Office
Office européen des brevets

(11) Veröffentlichungsnummer: **0 247 551 B1**

(12) # EUROPÄISCHE PATENTSCHRIFT

(45) Veröffentlichungstag der Patentschrift: **30.10.91**

(51) Int. Cl.$^5$: **C07D 237/16**, A01N 43/58

(21) Anmeldenummer: **87107542.0**

(22) Anmeldetag: **23.05.87**

(54) **Substituierte N-Phenylpyridazon-Derivate.**

(30) Priorität: **28.05.86 DE 3617997**

(43) Veröffentlichungstag der Anmeldung:
**02.12.87 Patentblatt 87/49**

(45) Bekanntmachung des Hinweises auf die Patenterteilung:
**30.10.91 Patentblatt 91/44**

(84) Benannte Vertragsstaaten:
**AT BE CH DE FR GB IT LI NL**

(56) Entgegenhaltungen:
**EP-A- 0 003 805**
**EP-A- 0 037 925**
**EP-A- 0 085 785**
**EP-A- 0 128 530**

(73) Patentinhaber: **BASF Aktiengesellschaft**
**Carl-Bosch-Strasse 38**
**W-6700 Ludwigshafen(DE)**

(72) Erfinder: **Becker, Rainer, Dr.**
**Im Haseneck 22**
**W-6702 Bad Duerkheim(DE)**
Erfinder: **Wriede, Ulrich, Dr.**
**Albert-Einstein-Allee 10**
**W-6703 Limburgerhof(DE)**
Erfinder: **Schirmer, Ulrich, Dr.**
**Berghalde 79**
**W-6900 Heidelberg(DE)**
Erfinder: **Parg, Adolf, Dr.**
**Paray-le-Monial-Strasse 8**
**W-6702 Bad Duerkheim(DE)**
Erfinder: **Wuerzer, Bruno, Dr.**
**Ruedigerstrasse 13**
**W-6701 Otterstadt(DE)**
Erfinder: **Meyer, Norbert, Dr.**
**Dossenheimer Weg 22**
**W-6802 Ladenburg(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte N-Phenylpyridazonderivate der allgemeinen Formel I

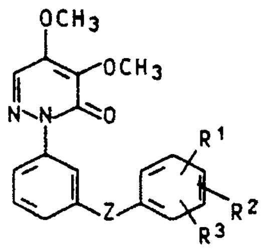

(I).

In dieser Formel bedeuten:

| | |
|---|---|
| $R^1$, $R^2$, $R^3$ | Wasserstoff, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Alkylsulfonyl, Halogen, Cyano, Phenyl, Phenoxy oder Phenylthio, |
| Z | eine unverzweigte, gesättigte Alkylengruppe mit 1 bis 5 C-Atomen. |

Außerdem betrifft die Erfindung die Herstellung der Verbindungen I und ihre Verwendung zur Bekämpfung unerwünschten Pflanzenwuchses, Mittel, die die Verbindungen I enthalten, sowie ein Verfahren zur Bekämpfung unerwünschten Pflanzenwuchses.

Aus der EP-A-128 530 sind strukturell ähnliche Verbindungen bekannt, die sich von den Verbindungen I dadurch unterscheiden, daß das Brückenglied zwischen den Phenylringen durch Heteroatome unterbrochen ist.

Obwohl die bekannten Verbindungen eine gute herbizide Wirkung zeigen, lag der Erfindung die Bereitstellung noch wirksamerer Substanzen zugrunde.

Demgemäß wurde gefunden, daß sich die eingangs definierten neuen substituierten N-Phenylpyridazonderivate I hervorragend zur Bekämpfung unerwünschten Pflanzenwuchses eignen und gegenüber Nutzpflanzen selektiv sind.

Die Verbindungen des Typs I sind durch die folgende Reaktionssequenz erhältlich:

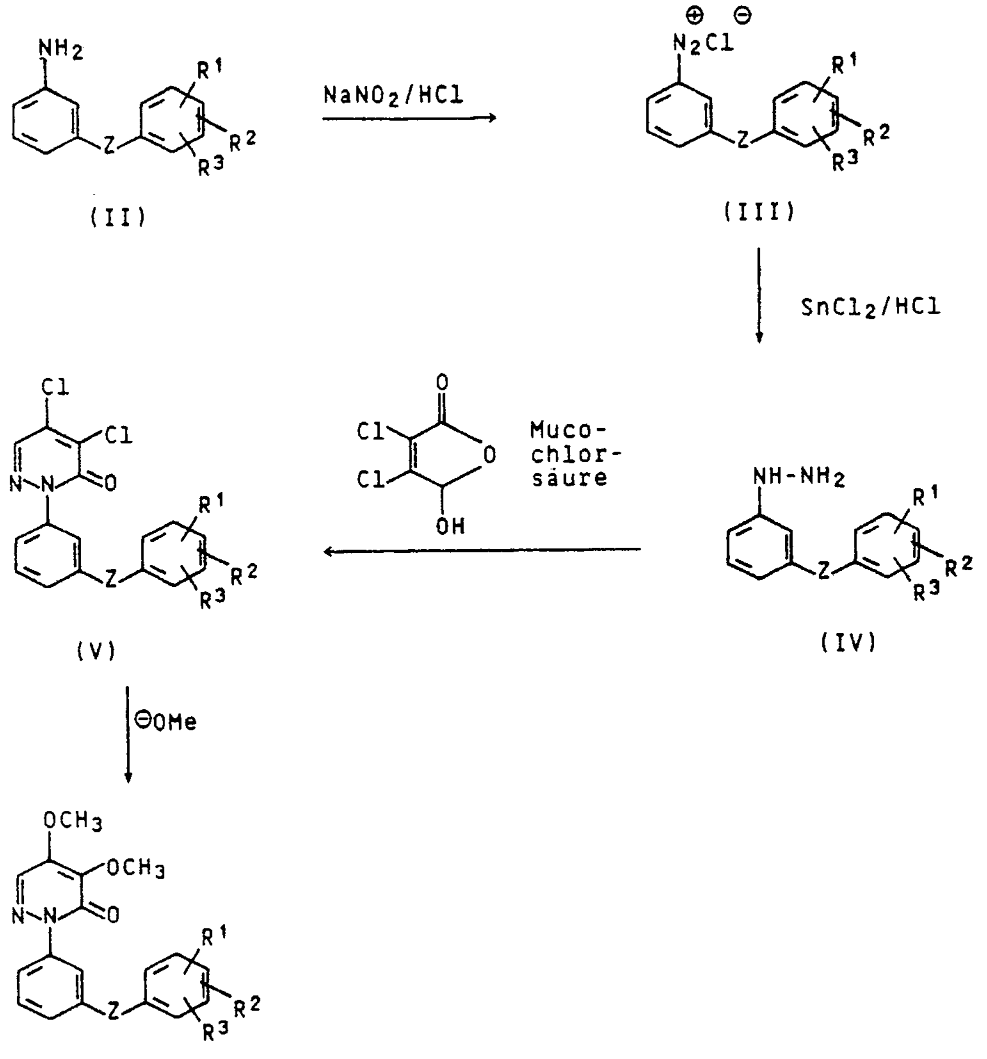


(II) (III) (IV) (V)

(I)

Zur Herstellung der Verbindungen II

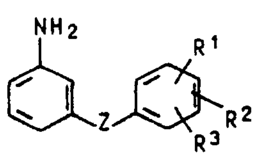

(II)

eignen sich für jede Kettenlänge von $C_1$ bis $C_5$ für Z unterschiedliche Verknüpfungsreaktionen besonders:

- Für den Fall, daß Z = $CH_2$ bedeutet, setzt man in an sich bekannter Weise 3-Nitrobenzoylchlorid mit einem Benzolderivat durch Friedel-Crafts-Acylierung zu den 3-Nitrobenzophenonen um,

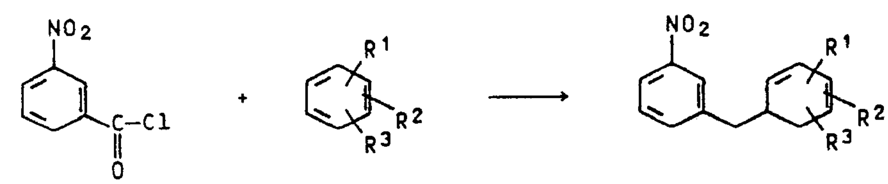

- für den Fall, daß Z = $CH_2CH_2$ bedeutet, wird in an sich bekannter Weise 3-Nitrobenzaldehyd mit Benzylphosphoniumhalogeniden in einer Wittigreaktion zu den 3-Nitrostilbenen umgesetzt,

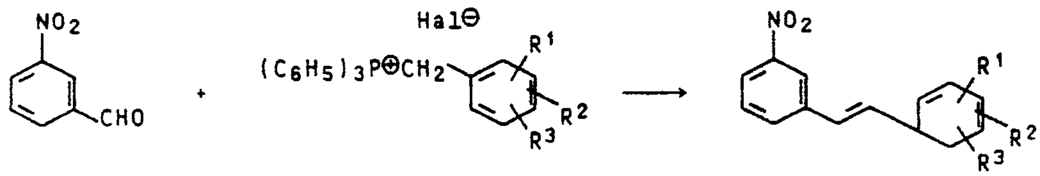

Hal = Halogen

- für den Fall, daß Z = $CH_2CH_2CH_2$ bedeutet, kann die Umsetzung in an sich bekannter Weise durch Aldolkondensation von 3-Nitroacetophenon und Benzaldehyden zu den 3-Nitrobenzalacetophenonen erfolgen,

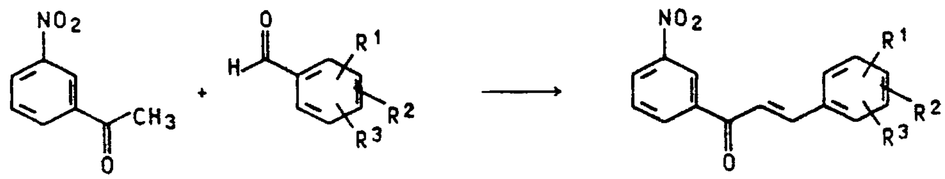

- für den Fall, daß Z = $CH_2CH_2CH_2CH_2$ bedeutet, läßt sich in an sich bekannter Weise eine Wittig-Reaktion zwischen 3-Nitrozimtaldehyd und einem Benzylphosphoniumhalogenid zu den 1-(3-Nitrophenyl)-3-phenylbutadienen durchführen,

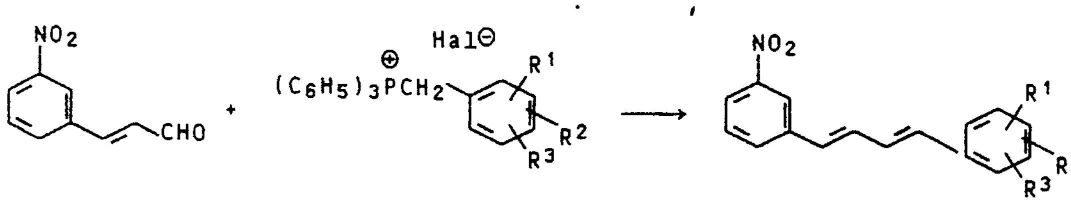

- für den Fall, daß Z = $CH_2CH_2CH_2CH_2CH_2$ bedeutet, kann man durch Aldolkondensation in an sich bekannter Weise 3-Nitroacetophenon und einen Zimtaldehyd zu den 3-Nitrocinamylidenacetophenonen umsetzen.

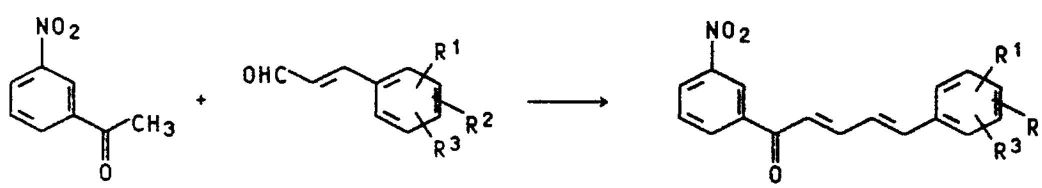

Anschließend werden die Nitroverbindungen - vorzugsweise katalytisch -und gegebenenfalls die ungesättigten Brückenglieder hydriert, wobei man einen üblichen Hydrierkatalysator (z.B. Palladium auf Kohle, Platinschwamm oder Raney-Nickel), ein geeignetes Lösungsmittel wie eine Carbonsäure (z.B. Essigsäure), Ether (z.B. Tetrahydrofuran) oder Alkohole (z.B. Ethanol) und gegebenenfalls eine starke Säure (z.B. Methansulfonsäure, Schwefelsäure) bei Temperaturen zwischen 0 und 150 °C sowie Drücken zwischen 1 und 100 bar anwendet (B.R. Baker et al, J.Pharm.Sci. 56, 737-742). Die entstehenden Aniline IV können ohne Isolierung der Zwischenstufen zu den substituierten N-Phenylpyridazonderivaten I über das Diazoniumsalz III und das Hydrazinderivat IV umgesetzt werden.

Im Fall der Isolierung/Reinigung der Hydrazinderivate IV, die man beispielsweise als Hydrochloride isolieren kann, erhält man reinere Endprodukte I.

Die Hydrazinderivate IV können aus den Anilinen II nach an sich bekannten Methoden durch Diazotierung und Reduktion (Houben/Weyl, Methoden der org. Chemie, Bd. 10/2, S. 180f, Georg-Thieme Verlag, 1967) hergestellt werden. Die Dihalogenpyridazone V lassen sich in an sich bekannter Weise (US-A-2 628 181 oder EP-A-128 530) durch Ringschluß mit Mucochlorsäure herstellen. Die erfindungsgemäßen substituierten N-Phenylpyridazonderivate I können schließlich durch Umsetzung mit Alkoholaten des Methanols, wie Natrium- und Kaliummethylat, bei Temperaturen zwischen 0 und 150° C, vorzugsweise 20 bis 120° C, in Gegenwart oder Abwesenheit eines zusätzlichen Lösungsmittels erhalten werden. Als Lösungsmittel sind Kohlenwasserstoffe beispielsweise Benzol, Toluol, Xylol, Pentan, Hexan, Heptan, Octan, Cyclopentan, Cyclohexan, Ether, z.B. Diethylether, Diisopropylether, Methyl-tert.-butylether, Tetrahydrofuran, Dioxan, Ethylenglykolether, Alkohole wie Methanol, Ethanol, n-, iso-Propanol, n-, iso-oder tert.-Butanol anwendbar; bevorzugt werden Benzol, Toluol und Xylol.

Die Reaktion kann im Druckbereich von 1 bis 15 bar, vorzugsweise bei 1 bar durchgeführt werden.

Die Reste $R^1$, $R^2$, $R^3$ in den Verbindungen I können außer Wasserstoff folgende Bedeutung haben:

- $C_1$-$C_4$-Alkyl, bevorzugt 2-, 3- oder 4-Methyl, 2-, 3- oder 4-Ethyl, 2-, 3- oder 4-iso-Propyl, 4-tert.-Butyl, 2,4-, 2,5-, 2,6-, 3,4-, 3,5-Dimethyl,
- $C_1$-$C_4$-Halogenalkyl, bevorzugt 2-, 3- oder 4-Trifluormethyl,
- $C_1$-$C_4$-Alkoxy, bevorzugt 2-, 3-, 4-Methoxy, 4-tert.-Butoxy, 3,4-Dimethoxy,
- $C_1$-$C_4$-Halogenalkoxy, bevorzugt 2-, 3- oder 4-Trifluormethoxy, 3- oder 4-[(1,1,2,2)-Tetrafluorethoxy],
- $C_1$-$C_4$-Alkylthio, bevorzugt 3-oder 4-Methylthio,
- $C_1$-$C_4$-Halogenalkylthio, bevorzugt 3- oder 4-Trifluormethylthio,
- $C_1$-$C_4$-Alkylsulfinyl, bevorzugt 3- oder 4-Methylsulfinyl,
- $C_1$-$C_4$-Alkylsulfonyl, bevorzugt 3- oder 4-Methylsulfonyl,
- Halogen, bevorzugt 2-, 3- oder 4-Fluor, 2-, 3- oder 4-Chlor, 3-oder 4-Brom, 2,4-, 2,5-, 2,6-, 3,4-oder 3,5-Dichlor, 2,3,4-Trichlor,
- Cyano, bevorzugt 4-Cyano,
- Phenyl, bevorzugt 3- und 4-Phenyl,
- Phenoxy, bevorzugt 3- und 4-Phenoxy und
- Phenylthio, bevorzugt 4-Phenylthio.

Bevorzugt werden u.a. solche Verbindungen I, in denen einer oder insbesondere zwei der Substituenten $R^1$, $R^2$ und $R^3$ für Wasserstoff stehen und Z eine unverzweigte, gesättigte Alkylengruppe mit 1 bis 3 C-Atomen bedeutet.

Beispiele für geeignete Verbindungen I sind:

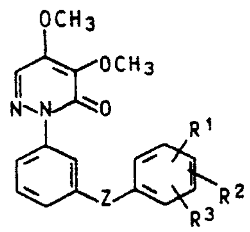

(I)

| Verbindung | Z | $R^1$, $R^2$, $R^3$ |
|---|---|---|
| 1 | $CH_2$ | H |
| 2 | $CH_2$ | 4-Cl |
| 3 | $CH_2$ | 4-Br |
| 4 | $CH_2$ | 4-$CH_3$ |
| 5 | $CH_2$ | 4-$OCH_3$ |
| 6 | $CH_2$ | 4-$SCH_3$ |
| 7 | $CH_2$ | 4-$SOCH_3$ |
| 8 | $CH_2$ | 4-$SO_2$-$CH_3$ |
| 9 | $CH_2$ | 2,5-Dichlor |
| 10 | $CH_2$ | 3,4-Dichlor |
| 11 | $CH_2$ | 2,4-Dimethyl |
| 12 | $CH_2$ | 2,5-Dimethyl |
| 13 | $CH_2$ | 3,4-Dimethyl |
| 14 | $CH_2$ | 4-Phenoxy |
| 15 | $CH_2$ | 4-Phenylthio |
| 16 | $CH_2CH_2$ | H |
| 17 | $CH_2CH_2$ | 2-$CH_3$ |
| 18 | $CH_2CH_2$ | 3-$CH_3$ |
| 19 | $CH_2CH_2$ | 4-$CH_3$ |
| 20 | $CH_2CH_2$ | 4-i-$C_3H_7$ |
| 21 | $CH_2CH_2$ | 4-t-$C_4H_9$ |
| 22 | $CH_2CH_2$ | 4-Phenyl |
| 23 | $CH_2CH_2$ | 2,4-Dimethyl |
| 24 | $CH_2CH_2$ | 2,5-Dimethyl |
| 25 | $CH_2CH_2$ | 3,4-Dimethyl |
| 26 | $CH_2CH_2$ | 2-$CF_3$ |
| 27 | $CH_2CH_2$ | 3-$CF_3$ |
| 28 | $CH_2CH_2$ | 4-$CF_3$ |
| 29 | $CH_2CH_2$ | 2-F |
| 30 | $CH_2CH_2$ | 3-F |

| Verbindung | Z | $R^1$, $R^2$, $R^3$ |
|---|---|---|
| 31 | $CH_2CH_2$ | 4-F |
| 32 | $CH_2CH_2$ | 2-Cl |
| 33 | $CH_2CH_2$ | 3-Cl |
| 34 | $CH_2CH_2$ | 4-Cl |
| 35 | $CH_2CH_2$ | 4-Br |
| 36 | $CH_2CH_2$ | 2,6-Dichlor |
| 37 | $CH_2CH_2$ | 2,4-Dichlor |
| 38 | $CH_2CH_2$ | 3,4-Dichlor |
| 39 | $CH_2CH_2$ | 3,5-Dichlor |
| 40 | $CH_2CH_2$ | 2,5-Dichlor |
| 41 | $CH_2CH_2$ | 2,3,4-Trichlor |
| 42 | $CH_2CH_2$ | 4-CN |
| 43 | $CH_2CH_2$ | 2-$OCH_3$ |
| 44 | $CH_2CH_2$ | 3-$OCH_3$ |
| 45 | $CH_2CH_2$ | 4-$OCH_3$ |
| 46 | $CH_2CH_2$ | 4-O-t-$C_4H_9$ |
| 47 | $CH_2CH_2$ | 3-Phenoxy |
| 48 | $CH_2CH_2$ | 4-Phenoxy |
| 49 | $CH_2CH_2$ | 3,4-Dimethoxy |
| 50 | $CH_2CH_2$ | 4-$SCH_3$ |
| 51 | $CH_2CH_2$ | 3-$OCF_2CHF_2$ |
| 52 | $CH_2CH_2$ | 4-$OCF_2CHF_2$ |
| 53 | $CH_2CH_2CH_2$ | H |
| 54 | $CH_2CH_2CH_2$ | 2-$CH_3$ |
| 55 | $CH_2CH_2CH_2$ | 3-$CH_3$ |
| 56 | $CH_2CH_2CH_2$ | 4-$CH_3$ |
| 57 | $CH_2CH_2CH_2$ | 4-i-$C_3H_7$ |
| 58 | $CH_2CH_2CH_2$ | 4-t-$C_4H_9$ |
| 59 | $CH_2CH_2CH_2$ | 4-Phenyl |
| 60 | $CH_2CH_2CH_2$ | 2,4-Dimethyl |
| 61 | $CH_2CH_2CH_2$ | 3,4-Dimethyl |
| 62 | $CH_2CH_2CH_2$ | 3,5-Dimethyl |
| 63 | $CH_2CH_2CH_2$ | 2-F |
| 64 | $CH_2CH_2CH_2$ | 3-F |
| 65 | $CH_2CH_2CH_2$ | 4-F |
| 66 | $CH_2CH_2CH_2$ | 2-Cl |
| 67 | $CH_2CH_2CH_2$ | 3-Cl |
| 68 | $CH_2CH_2CH_2$ | 4-Cl |
| 69 | $CH_2CH_2CH_2$ | 2,6-Dichlor |
| 70 | $CH_2CH_2CH_2$ | 2,5-Dichlor |
| 71 | $CH_2CH_2CH_2$ | 3,4-Dichlor |

| Verbindung | Z | $R^1$, $R^2$, $R^3$ |
|---|---|---|
| 72 | $CH_2CH_2CH_2$ | 3,5-Dichlor |
| 73 | $CH_2CH_2CH_2$ | 2,4-Dichlor |
| 74 | $CH_2CH_2CH_2$ | 4-Br |
| 75 | $CH_2CH_2CH_2$ | 2-$CF_3$ |
| 76 | $CH_2CH_2CH_2$ | 3-$CF_3$ |
| 77 | $CH_2CH_2CH_2$ | 4-$CF_3$ |
| 78 | $CH_2CH_2CH_2$ | 2-$OCH_3$ |
| 79 | $CH_2CH_2CH_2$ | 3-$OCH_3$ |
| 80 | $CH_2CH_2CH_2$ | 4-$OCH_3$ |
| 81 | $CH_2CH_2CH_2$ | 3,4-Dimethoxy |
| 82 | $CH_2CH_2CH_2$ | 4-O-t-$C_4H_9$ |
| 83 | $CH_2CH_2CH_2$ | 3-Phenoxy |
| 84 | $CH_2CH_2CH_2$ | 4-Phenoxy |
| 85 | $CH_2CH_2CH_2$ | 4-$SCH_3$ |
| 86 | $CH_2CH_2CH_2$ | 3-$OCF_2CHF_2$ |
| 87 | $CH_2CH_2CH_2$ | 4-$OCF_2CHF_2$ |
| 88 | $CH_2CH_2CH_2$ | 4-CN |
| 89 | $CH_2CH_2CH_2CH_2$ | H |
| 90 | $CH_2CH_2CH_2CH_2$ | 3-$CH_3$ |
| 91 | $CH_2CH_2CH_2CH_2$ | 4-$CH_3$ |
| 92 | $CH_2CH_2CH_2CH_2$ | 4-t-$C_4H_9$ |
| 93 | $CH_2CH_2CH_2CH_2$ | 2,4-Dimethyl |
| 94 | $CH_2CH_2CH_2CH_2$ | 3-F |
| 95 | $CH_2CH_2CH_2CH_2$ | 4-F |
| 96 | $CH_2CH_2CH_2CH_2$ | 2-Cl |
| 97 | $CH_2CH_2CH_2CH_2$ | 3-Cl |
| 98 | $CH_2CH_2CH_2CH_2$ | 4-Cl |
| 99 | $CH_2CH_2CH_2CH_2$ | 2,4-Dichlor |
| 100 | $CH_2CH_2CH_2CH_2$ | 3,5-Dichlor |
| 101 | $CH_2CH_2CH_2CH_2$ | 3,4-Dichlor |
| 102 | $CH_2CH_2CH_2CH_2$ | 3-$CF_3$ |
| 103 | $CH_2CH_2CH_2CH_2$ | 4-$CF_3$ |
| 104 | $CH_2CH_2CH_2CH_2$ | 3-$OCH_3$ |
| 105 | $CH_2CH_2CH_2CH_2$ | 4-$OCH_3$ |
| 106 | $CH_2CH_2CH_2CH_2$ | 4-$SCH_3$ |
| 107 | $(CH_2)_5$ | H |
| 108 | $(CH_2)_5$ | 4-Cl |
| 109 | $CH_2$ | 4-F |
| 110 | $CH_2CH_2CH_2$ | 2,3,4-Trichlor |
| 111 | $(CH_2)_5$ | 4-F |

Die substituierten N-Phenylpyridazonderivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder

Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentrate n, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Lauryl alkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 19 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 53 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 30 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 29 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteile n der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 37 werden mit 3 Gewichtsteilen des Natriumsalzes der

Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 3 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 33 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 44 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. 40 Gew.-Teile des Wirkstoffs Nr. 65 werden in 60 Gewichtsteilen einer Mischung, die aus 93 Gew.% Xylol und 7 Gew.% des Anlagerungsproduktes von 8 Mol Ethylenoxid an 1 Mol Nonylphenol besteht, gelöst. Man erhält eine Lösung, die 40 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 5,0, vorzugsweise 0,05 bis 0,5 kg/ha.

In Anbetracht des erfaßbaren Wirkungsspektrums zur Unkrautbekämpfung, der Verträglichkeit der Kulturpflanzen oder der erwünschten Beeinflussung des Wachstums derselben sowie angesichts der Vielfalt der Applikationsmethoden können die erfindungsgemäßen Verbindungen je nach Substitutionsmuster in einer großen Zahl von Kulturpflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |

| Botanischer Name | Deutscher Name |
|---|---|
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |

| Botanischer Name | Deutscher Name |
|---|---|
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die substituierten N-Phenylpyridazonderivate der allgemeinen Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die neuen Verbindungen der Formel I allein oder in Kombination mit anderen Herbiziden, auch noch mit weiteren Pflanzenschutzmitteln gemischt, gemeinsam aus zubringen, beispielsweise mit Mitteln zur Bekämpfung von Schädlingeen oder phytopatogenen Pilzen bzw. Bakterien. Von Interesse ist ferner die Mischbarkeit mit Mineralsalzlösungen, welche zur Behe bung von Ernährungs- und Spurenelementmängeln eingesetzt werden. Es können auch nichtphytotoxische Öle und Ölkonzentrate hergestellt werden.

## Herstellungsbeispiele

### Herstellung der Verbindungen I

192 mmol eines Anilins II, gelöst in 290 ml Eisessig und 68 ml konz. Salzsäure, wurden bei 0 bis 7° C tropfenweise mit einer Lösung von 13,5 g Natriumnitrit in 30 ml Wasser versetzt. Nach einstündigem Nachrühren bei 5° C tropfte man eine Lösung von 58 g Zinn-II-chlorid-Dihydrat in 77 ml konz. Salzsäure zu, wobei die Temperatur 5° C nicht überstieg. Anschließendes Erwärmen auf 20° C, 30 minütiges Nachrühren und Zugabe von 32 g Mucochlorsäure innerhalb von 5 Min. und 5-minütiges Erhitzen zum Rückfluß ergab eine Reaktionsmischung, die in Eiswasser gegeben und mit Methylenchlorid extrahiert wurde. Nach dem Waschen der organischen Phase mit Wasser wurde getrocknet und eingeengt. Das Rohprodukt reinigte man durch Chromatographie an Kieselgel mit Methylenchlorid/Pentan als Laufmittel.

65 mmol eines Dichlorpyridazon V in 300 ml Toluol versetzte man mit 110 g Natriummethylat und rührte 2 bis 4 Stunden bei 60 bis 75° C. Es wurden 400 ml Methylenchlorid zugesetzt und die Lösung dreimal mit Wasser gewaschen, getrocknet und eingeengt. Das Rohprodukt reinigte man durch Chromatographie an Kieselgel mit Methyl-tert.-butylether/Pentan als Laufmittel. Die Ergebnisse sind in der folgenden Tabelle zusammengestellt.

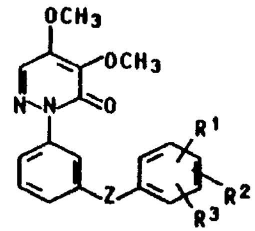

(I)

| Verbindung Nr. | Z | $R^1$, $R^2$, $R^3$ | Fp [°C] | IR [$cm^{-1}$] | $^1H$- NMR [ppm] |
|---|---|---|---|---|---|
| 1 | $CH_2$ | H | 105-106 | | |
| 2 | $CH_2$ | 4-Cl | 72-73 | | |
| 3 | $CH_2$ | 4-Br | | | 8,20 (s, 1H) |
| 4 | $CH_2$ | 4-$CH_3$ | 52-53 | | |
| 5 | $CH_2$ | 4-$OCH_3$ | | 1652 (C=O) | |
| 6 | $CH_2$ | 4-$SCH_3$ | | | 8,19 (s, 1H) |
| 13 | $CH_2$ | 3,4-Dimethyl | | 1653 (C=O) | |
| 16 | $CH_2CH_2$ | H | 78-81 | | |
| 17 | $CH_2CH_2$ | 2-$CH_3$ | 85-87 | | |
| 18 | $CH_2CH_2$ | 3-$CH_3$ | 68-70 | | |
| 19 | $CH_2CH_2$ | 4-$CH_3$ | 100-102 | | |
| 21 | $CH_2CH_2$ | 4-t-$C_4H_9$ | | | 7,83 (s, 1H) |
| 27 | $CH_2CH_2$ | 3-$CF_3$ | 89-90 | | |
| 28 | $CH_2CH_2$ | 4-$CF_3$ | 96-97 | | |
| 29 | $CH_2CH_2$ | 2-F | 116-118 | | |
| 30 | $CH_2CH_2$ | 3-F | 60-61 | | |
| 31 | $CH_2CH_2$ | 4-F | 78-80 | | |
| 33 | $CH_2CH_2$ | 3-Cl | 58-60 | | |
| 34 | $CH_2CH_2$ | 4-Cl | 87-88 | | |
| 36 | $CH_2CH_2$ | 2,6-Dichlor | 89-91 | | |
| 37 | $CH_2CH_2$ | 2,4-Dichlor | 80-82 | | |

| Verbindung Nr. | Z | $R^1$, $R^2$, $R^3$ | Fp [°C] | IR [$cm^{-1}$] | $^1H$-NMR [ppm] |
|---|---|---|---|---|---|
| 38 | $CH_2CH_2$ | 3,4-Dichlor | 74-76 | | |
| 40 | $CH_2CH_2$ | 2,5-Dichlor | 102-103 | | |
| 44 | $CH_2CH_2$ | 3-$OCH_3$ | | 1652 (C=O) | |
| 45 | $CH_2CH_2$ | 4-$OCH_3$ | 68-70 | | |
| 50 | $CH_2CH_2$ | 4-$SCH_3$ | 68-69 | | |
| 53 | $CH_2CH_2CH_2$ | H | 73-75 | | |
| 54 | $CH_2CH_2CH_2$ | 2-$CH_3$ | 38-40 | | |
| 55 | $CH_2CH_2CH_2$ | 3-$CH_3$ | 47-49 | | |
| 56 | $CH_2CH_2CH_2$ | 4-$CH_3$ | 68-70 | | |
| 57 | $CH_2CH_2CH_2$ | 4-i-$C_3H_7$ | 55-56 | | |
| 58 | $CH_2CH_2CH_2$ | 4-t-$C_4H_9$ | | 1654 (C=O) | |
| 59 | $CH_2CH_2CH_2$ | 4-Phenyl | | 1652 (C=O) | |
| 60 | $CH_2CH_2CH_2$ | 2,4-Dimethyl | 50-50,5 | | |
| 63 | $CH_2CH_2CH_2$ | 2-F | 60-61 | | |
| 64 | $CH_2CH_2CH_2$ | 3-F | | 1653 (C=O) | |
| 65 | $CH_2CH_2CH_2$ | 4-F | 61-63 | | |
| 67 | $CH_2CH_2CH_2$ | 3-Cl | 53-55 | | |
| 68 | $CH_2CH_2CH_2$ | 4-Cl | | | 7,83 (s, 1H) |
| 69 | $CH_2CH_2CH_2$ | 2,6-Dichlor | 107-109 | | |
| 70 | $CH_2CH_2CH_2$ | 2,5-Dichlor | 86-88 | | |
| 71 | $CH_2CH_2CH_2$ | 3,4-Dichlor | | 1651 (C=O) | |
| 72 | $CH_2CH_2CH_2$ | 3,5-Dichlor | | 1652 (C=O) | |
| 73 | $CH_2CH_2CH_2$ | 2,4-Dichlor | 118-119 | | |
| 76 | $CH_2CH_2CH_2$ | 3-$CF_3$ | 81-83 | | |

| Verbindung Nr. | Z | $R^1$, $R^2$, $R^3$ | Fp [°C] | IR [$cm^{-1}$] | $^1H$-NMR [ppm] |
|---|---|---|---|---|---|
| 77 | $CH_2CH_2CH_2$ | 4-$CF_3$ | | 1633 (C=O) | |
| 79 | $CH_2CH_2CH_2$ | 3-$OCH_3$ | 96-98 | | |
| 80 | $CH_2CH_2CH_2$ | 4-$OCH_3$ | | 1652 (C=O) | |
| 81 | $CH_2CH_2CH_2$ | 3,4-Dimethoxy | | 1653 (C=O) | |
| 83 | $CH_2CH_2CH_2$ | 3-Phenoxy | | | 7,82 (s, 1H) |
| 86 | $CH_2CH_2CH_2$ | 3-$OCF_2CHF_2$ | 40-45 | | |
| 87 | $CH_2CH_2CH_2$ | 4-$OCF_2CHF_2$ | | 1652 (C=O) | |
| 89 | $CH_2CH_2CH_2CH_2$ | H | | 1653 (C=O) | |
| 107 | $(CH_2)_5$ | H | | 1652 (C=O) | |
| 109 | $CH_2$ | 4-F | 108-110 | | |
| 110 | $CH_2CH_2CH_2$ | 2,3,4-Trichlor | 83-85 | | |
| 111 | $(CH_2)_5$ | 4-F | | | 8,23 (s, 1H) |

Anwendungsbeispiele

Die herbizide Wirkung der N-Phenylpyridazone der Formel I auf das Wachstum der Testpflanzen bei Nachauflaufanwendung wurde durch folgende Gewächshausversuche gezeigt:

Als Kulturgefäße dienten Plastikblumentöpfe mit 300 $cm^3$ Inhalt und lehmigem Sand mit etwa 3,0 % Humus als Substrat. Bei Soja wurde etwas Torfmull zugesetzt, um einen besseren Stand zu erzielen. Die Samen der Testpflanzen wurden nach Arten getrennt flach eingesät.

Zum Zwecke der Nachauflaufbehandlung wurden entweder direkt gesäte oder in den gleichen Gefäßen aufgewachsene Pflanzen ausgewählt oder erst als Keimpflanzen getrennt angezogen und einige Tage vor der Behandlung in die Versuchsgefäße verpflanzt.

Je nach Wuchsform wurden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt wurden, behandelt. Die Aufwandmengen für die Nachauflaufbehandlung variierten; sie betrugen 0,06 bis 0,25 kg Wirkstoff/ha.

Die Versuchsgefäße wurden im Gewächshaus aufgestellt, wobei für wärmeliebende Arten wärmere Bereiche (20 bis 35 °C) und für solche gemäßigter Klimate 10 bis 20 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet.

Folgende Pflanzen werden auf ihr Verhalten gegenüber den Verbindungen I sowie gegenüber bekannten Verbindungen untersucht:

| Lateinischer Name | Deutscher Name |
|---|---|
| Abutilon theophrasti | Chinesischer Hanf |
| Amaranthus retro flexus | zurückgekrümmter Fuchsschwanz |
| Arachis hypogaea | Erdnuß |
| Cassia tora | - |
| Centaurea cyanus | Kornblume |
| Chenopodium album | Weißer Gänsefuß |
| Chrysanthemum coronarium | Kronenwucherblume (Goldblume) |
| Desmodium tortuosum | - |
| Euphorbia heterophylla | Wolfsmilchart |
| Galium aparine | Klettenlabkraut |
| Glycine max. | Sojabohne |

| Lateinischer Name | Deutscher Name |
|---|---|
| Ipomoea spp. | Prunkwindearten |
| Lamium amplexicaule | Stengelumfassende Taubnessel |
| Mercurialis annua | Einjähriges Bindelkraut |
| Polygonum persicaria | Flohknöterich |
| Stellaria media | Vogelsteinmiere |
| Solanum nigrum | Schwarzer Nachtschatten |
| Triticum aestivum | Weizen |
| Zea mays | Mais |

Als Vergleichssubstanzen I′ wurden Wirkstoffe aus EP-A-128 530, in denen Z′ eine sauerstoff- oder schwefelhaltige Kette bedeutet, verwendet und den neuen Wirkstoffen mit gleicher Kettenlänge von Z gegenübergestellt.

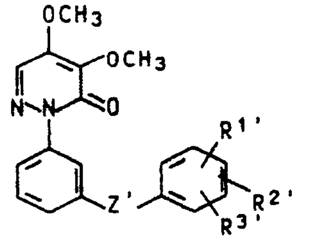

(I')

| Vergleichssubstanzen I' | Z' | R1', R2', R3' | Bsp. in EP-A-128 530 |
|---|---|---|---|
| A | $CH_2S$ | 4-$CH_3$ | 114 |
| B | $CH_2O$ | 4-$CH_3$ | 24 |
| C | $CH_2O$ | H | 1 |
| D | $SCH_2$ | H | 126 |
| E | $CH_2S$ | H | 105 |
| F | $CH_2OCH_2$ | H | 102 |
| G | $OCH_2CH_2$ | H | 87 |
| H | $CH_2O$ | 3-F | 3 |
| I | $OCH_2$ | 3-F | 64 |
| K | $CH_2O$ | 2,4-Dichlor | 13 |
| L | $OCH_2$ | 3-Chlor | 67 |
| M | $CH_2O$ | 4-$SCH_3$ | 46 |

Bei einer Aufwandmenge von 0,06 kg Wirkstoff/ha ließen sich mit Verbindung 19 dikotyle unerwünschte Pflanzen bei guter Verträglichkeit für Erdnuß deutlich besser bekämpfen als mit Vergleichssubstanz A.

Zur selektiven Bekämpfung dikotyler Pflanzen eignete sich bei einer Aufwandmenge von 0,06 g Wirkstoff/ha Verbindung 19 besser als die bekannten Wirkstoffe A und B. In der Verträglichkeit für Weizen unterschieden sich die Wirkstoffe nur unwesentlich.

Ein breites Spektrum unerwünschter dikotyler Pflanzen wurde bei einer Aufwandmenge von 0,06 kg Wirkstoff/ha von der Verbindung 16 erfaßt. Mais als monokotyle Kulturpflanze erfuhr keine nennenswerte Schädigung. Die Vergleichssubstanzen C, D, E wirkten deutlich schwächer herbizid und verursachten darüber hinaus deutliche Schäden an der Nutzpflanze.

Mit Verbindung 53 konnten mit 0,125 kg Wirkstoff/ha dikotyle Schadpflanzen gut kontrolliert werden; Erdnußpflanzen wurden nicht beeinträchtigt. Das herbizide Wirkniveau der Vergleichssubstanz F lag deutlich niedriger.

Zur Bekämpfung dikotylen unerwünschten Pflanzenwuchses mit 0,125 kg Wirkstoff/ha wurde Verbindung 53 einsetzt, ohne die Kulturpflanze Weizen nennenswert zu schädigen. Die Wirkstoffe F und G zeigten bei Anwendung der gleichen Menge deutlich schwächere herbizide Eigenschaften.

Ein breites Spektrum unerwünschter Pflanzen wurde bei einer Aufwandmenge von 0,06 kg Wirkstoff/ha mit Verbindung 30 gut kontrolliert. Das herbizide Niveau der Vergleichssubstanzen H und I lag deutlich niedriger. Die Kulturpflanze Weizen erlitt keine Schädigung beim Einsatz von Verbindung 30, H und I. Im Falle von Mais als Kulturpflanze schädigten die Verbindungen 30 und H diese leicht; Verbindung I bewirkte eine stärkere Schädigung von Mais.

Mit Verbindung 37 konnten mit 0,125 kg Wirkstoff/ha unerwünschte Pflanzen besser bekämpft werden als mit dem Wirkstoff K; Erdnußpflanzen wurden durch beide Wirkstoffe nicht nennenswert geschädigt.

Ein breites Spektrum unerwünschter Pflanzen wurde bei einer Aufwandmenge von 0,06 kg Wirkstoff/ha mit Verbindung 33 deutlich besser bekämpft als mit der Vergleichsverbindung L.

Mit einer Aufwandmenge von 0,5 kg Wirkstoff/ha ließen sich mit Verbindung 50 unerwünschte breitblättrige Pflanzen gut bekämpfen ohne Soja zu schädigen. Der Wirkstoff M zeigte bei Anwendung der gleichen Menge deutlich schwächere herbizide Aktivität und schädigte Soja leicht.

Mit Aufwandmengen von 0,125 bis 0,25 kg Wirkstoff/ha ließen sich unerwünschte Pflanzen mit den

Verbindungen 29, 44 und 65 gut bekämpfen. Soja als Kulturpflanze wurde nur geringfügig beeinflußt.

Wichtige Problemunkräuter ließen sich mit Aufwandmengen zwischen 0,06 und 0,25 kg Wirkstoff/ha mit den Verbindungen 3, 17, 29, 59, 71 und 77 gut bekämpfen. Die Kulturpflanze Weizen wurde dabei allenfalls unwesentlich geschädigt.

Ein breites Spektrum breitblättriger unerwünschter Pflanzen ließ sich mit Aufwandmengen zwischen 0,06 und 0,25 kg Wirkstoff/ha mit den Verbindungen 19, 28, 31, 34, 38 und 54 gut bekämpfen.

## Patentansprüche

1. Substituierte N-Phenylpyridazonderivate der allgemeinen Formel I

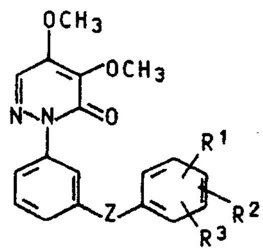

(I)

in der die Substituenten folgende Bedeutung haben:

| | |
|---|---|
| $R^1$, $R^2$, $R^3$ | Wasserstoff, $C_1$-$C_4$-Alkyl, $C_1$-$C_4$-Halogenalkyl, $C_1$-$C_4$-Alkoxy $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Alkylsulfonyl, Halogen, Cyano, Phenyl, Phenoxy oder Phenylthio, |
| Z | eine unverzweigte, gesättigte Alkylengruppe mit 1 bis 5 C-Atomen. |

2. Substituiertes N-Phenylpyridazonderivat der allgemeinen Formel I nach Anspruch 1, in der

| | |
|---|---|
| $R^1$, $R^2$, $R^3$ | Wasserstoff, $C_1$-$C_4$-Alkyl, $C_1$-$C_4$-Halogenalkyl, $C_1$-$C_4$-Alkoxy, $C_1$-$C_4$-Alkylthio, Halogen oder Phenyl und |
| Z | eine unverzweigte, gesättigte Alkylengruppe mit 1 bis 3 C-Atomen |

bedeutet.

3. Substituierte N-Phenylpyridazonderivate der allgemeinen Formel I nach Anspruch 1, in der

| | |
|---|---|
| $R^1$ | Wasserstoff, |
| $R^2$, $R^3$ | Wasserstoff, $C_1$-$C_4$-Alkyl, $C_1$-$C_4$-Halogenalkyl, $C_1$-$C_4$-Alkoxy, $C_1$-$C_4$-Alkylthio, Halogen oder Phenyl und |
| Z | eine unverzweigte, gesättigte Alkylengruppe mit 1 bis 3 C-Atomen |

bedeutet.

4. Substituierte N-Phenylpyridazonderivate der allgemeinen Formel I nach Anspruch 1, in der

| | |
|---|---|
| $R^1$, $R^2$ | Wasserstoff, |
| $R^3$ | Wasserstoff, $C_1$-$C_4$-Alkyl, $C_1$-$C_4$-Halogenalkyl, $C_1$-$C_4$-Alkoxy, $C_1$-$C_4$-Alkylthio, Halogen oder Phenyl und |
| Z | eine unverzweigte, gesättigte Alkylengruppe mit 1 bis 3 C-Atomen |

bedeutet.

5. Verfahren zur Herstellung der substituierten N-Phenylpyridazonderivate I, in der

| | |
|---|---|
| $R^1$, $R^2$, $R^3$ | Wasserstoff, $C_1$-$C_4$-Alkyl, $C_1$-$C_4$-Halogenalkyl, $C_1$-$C_4$-Alkoxy $C_1$-$C_4$-Halogenalkoxy, $C_1$-$C_4$-Alkylthio, $C_1$-$C_4$-Halogen alkylthio, $C_1$-$C_4$-Alkylsulfinyl, $C_1$-$C_4$-Alkylsulfonyl, Halo- |

gen, Cyano, Phenyl, Phenoxy oder Phenylthio,

Z eine unverzweigte, gesättigte Alkylengruppe mit 1 bis 5 C-Atomen, bedeuten, dadurch gekennzeichnet, daß man

a) eine Aminoverbindung II

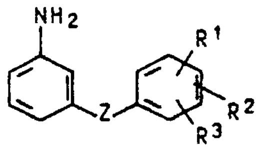

(II)

in an sich bekannter Weise zu der Verbindung III diazotiert

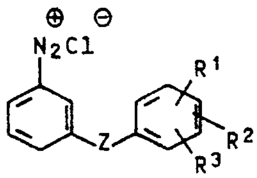

(III),

b) die Diazoniumverbindung III durch Reduktion in an sich bekannter Weise in die Hydrazinderivate IV überführt,

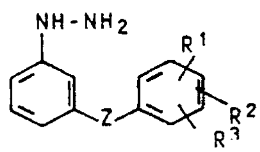

(IV),

c) die Hydrazinverbindung IV in an sich bekannter Weise mit Mucochlorsäure zum Pyridazonderivat V

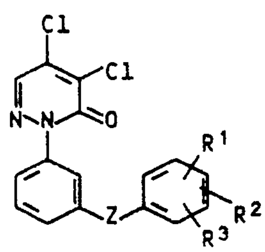

(V)

umsetzt und

d) die Pyridazinonverbindung V mit einem Methylat in an sich bekannter Weise in die substituierten N-Phenylpyridazonderivate I überführt.

**6.** Verwendung der substituierten N-Phenylpyridazonderivate I gemäß Anspruch 1 zur Bekämpfung unerwünschten Pflanzenwuchses.

**7.** Herbizid, enthaltend ein substituiertes N-Phenylpyridazonderivat I gemäß Anspruch 1.

**8.** Herbizid, enthaltend inerte Zusatzstoffe und ein substituiertes N-Phenylpyridazonderivat I gemäß Anspruch 1.

9. Verfahren zur Bekämpfung unerwünschten Pflanzenwuchses, dadurch gekennzeichnet, daß man die unerwünschten Pflanzen mit einer herbizid wirksamen Menge eines substituierten N-Phenylpyridazonderivates I gemäß Anspruch 1 behandelt.

## Claims

1. A substituted N-phenylpyridazone derivative of the general formula I

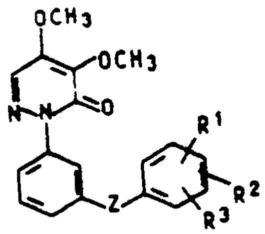

(I)

where $R^1$, $R^2$ and $R^3$ are each hydrogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, $C_1$-$C_4$-alkylsulfinyl, $C_1$-$C_4$-alkylsulfonyl, halogen, cyano, phenyl, phenoxy or phenylthio, and Z is a straight-chain, saturated alkylene group of 1 to 5 carbon atoms.

2. A substituted N-phenylpyridazone derivative of the general formula I as claimed in claim 1, where $R^1$, $R^2$ and $R^3$ are each hydrogen, $C_1$-$C_4$-alkyl, $C_1$-$C_4$-haloalkyl, $C_1$-$C_4$-alkoxy, $C_1$-$C_4$-alkylthio, halogen or phenyl, and Z is a straight-chain, saturated alkylene group of 1 to 3 carbon atoms.

3. A substituted N-phenylpyridazone derivative of the general formula I as claimed in claim 1, where $R^1$ is hydrogen, $R^2$ and $R^3$ are each hydrogen $C_1$-$C_4$-alkyl, $C_1$-$C_4$-haloalkyl, $C_1$-$C_4$-alkoxy, $C_1$-$C_4$-alkylthio, halogen or phenyl, and Z is a straight-chain, saturated alkylene group of 1 to 3 carbon atoms.

4. A substituted N-phenylpyridazone derivative of the general formula I as claimed in claim 1, where $R^1$ and $R^2$ are each hydrogen, $R^3$ is hydrogen, $C_1$-$C_4$-alkyl, $C_1$-$C_4$-haloalkyl, $C_1$-$C_4$-alkoxy, $C_1$-$C_4$-alkylthio, halogen or phenyl, and Z is a straight-chain, saturated alkylene group of 1 to 3 carbon atoms.

5. A process for the preparation of a substituted N-phenylpyridazone derivative I, where $R^1$, $R^2$ and $R^3$ are each hydrogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, $C_1$-$C_4$-alkylsulfinyl, $C_1$-$C_4$-alkylsulfonyl, halogen, cyano, phenyl, phenoxy or phenylthio, and Z is a straight-chain, saturated alkylene group of 1 to 5 carbon atoms, wherein

a) an amino compound II

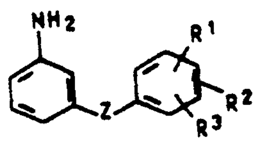

(II)

is diazotized in a conventional manner to give a compound III

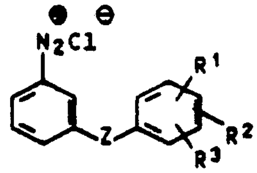

(III)

b) the diazonium compound III is converted in a conventional manner by reduction to give a hydrazine derivative IV

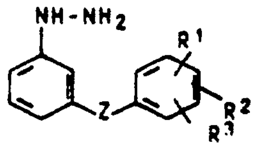

(IV)

c) the hydrazine compound IV is reacted in a conventional manner with mucochloric acid to give a pyridazone derivative V

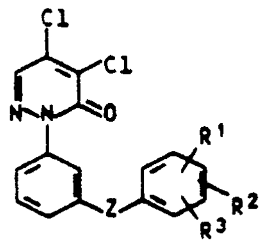

(V)

and

d) the pyridazone compound V is converted with a methylate in a conventional manner into a substituted N-phenylpyridazone derivative I.

6. Use of a substituted N-phenylpyridazone derivative I as claimed in claim 1 for controlling undesirable plant growth.

7. A herbicide containing a substituted N-phenylpyridazone derivative I as claimed in claim 1.

8. A herbicide containing inert additives and a substituted N-phenylpyridazone derivative I as claimed in claim 1.

9. A method for controlling the growth of undesirable plants, wherein the undesirable plants are treated with a herbicidally effective amount of a substituted N-phenylpyridazone derivative I claimed in claim 1.

**Revendications**

1. Dérivés substitués de N-phénylpyridazone de formule générale I

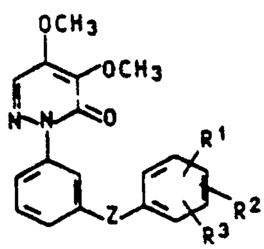

(I)

dans laquelle les substituants ont les significations suivantes :

| | |
|---|---|
| $R^1$, $R^2$ et $R^3$ | représentent l'hydrogène, des groupes alkyle en C 1-C 4, halogénoalkyle en C 1-C 4, alcoxy en C 1-C 4, halogénoalcoxy en C 1-C 4, alkylthio en C 1-C 4, halogénoalkylthio en C 1-C 4, alkylsulfinyle en C 1-C 4, alkylsulfonyle en C 1-C 4, des halogènes, des groupes cyano, phényle, phénoxy ou phénylthio, |
| Z | représente un groupe alkylène saturé non ramifié en C 1-C 5. |

2. Dérivé substitué de N-phénylpyridazone de formule générale I selon la revendication 1, dans laquelle

$R^1$, $R^2$ et $R^3$ représentent l'hydrogène, des groupes alkyle en C 1-C 4, halogénoalkyle en C 1-C 4, alcoxy en C 1-C 4, alkylthio en C 1-C 4, des halogènes ou des groupes phényle, et

Z représente un groupe alkylène saturé non ramifié en C 1-C 3.

**3.** Dérivés substitués de N-phénylpyridazone de formule générale I de la revendication 1, dans laquelle

$R^1$ représente l'hydrogène,

R2 et $R^3$ représentent l'hydrogène, des groupes alkyle en C 1-C 4, halogénoalkyle en C 1-C 4, alcoxy en C 1-C 4, alkylthio en C 1-C 4, des halogènes ou des groupes phényle, et

Z représente un groupe alkylène saturé non ramifié en C 1-C 3.

**4.** Dérivés substitués de N-phénylpyridazone de formule générale I de la revendication 1, dans laquelle

$R^1$ et $R^2$ représentent l'hydrogène,

$R^3$ représente l'hydrogène, un groupe alkyle en C 1-C 4, halogénoalkyle en C 1-C 4, alcoxy en C 1-C 4, alkylthio en C 1-C 4, un halogène ou un groupe phényle, et

Z représente un groupe alkylène saturé non ramifié en C 1-C 3.

**5.** Procédé de préparation des dérivés substitués de N-phénylpyridazone de formule I dans laquelle

$R^1$, $R^2$ et $R^3$ représentent l'hydrogène, des groupes alkyle en C 1-C 4, halogénolkyle en C 1-C 4, alcoxy en C 1-C 4, halogénoalcoxy en C 1-C 4, alkylthio en C 1-C 4, halogénoalkylthio en C 1-C 4, alkylsulfinyle en C 1-C 4, alkylsulfonyle en C 1-C 4, des halogènes, des groupes cyano, phényle, phénoxy ou phénylthio,

Z représente un groupe alkylène saturé non ramifié en C 1-C 5, caractérisé en ce que :

a) on diazote un dérivé aminé II

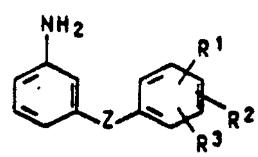

(II)

de manière connue en soi, ce qui donne le composé III

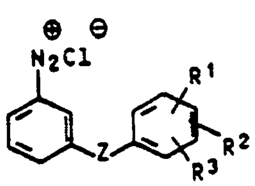

(III)

b) on convertit le dérivé de diazonium III par réduction, de manière connue en soi, en le dérivé d'hydrazine IV

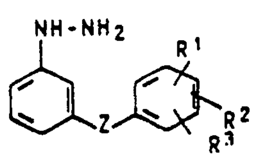

(IV)

c) on fait réagir le dérivé d'hydrazine IV, de manière connue en soi, avec l'acide mucochlorique, ce

qui donne le dérivé de pyridazone V

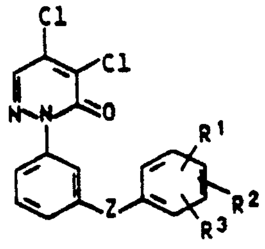

(V)

et

d) on convertit le dérivé de puridazone V, par réaction avec un méthylate, de manière connue en soi, en les dérivés substitués de N-phénylpyridazone I.

**6.** Utilisation des dérivés substitués de N-phénylpyridazone I selon la revendication 1 pour la lutte contre la croissance de végétaux indérisables.

**7.** Produit herbicide contenant un dérivé substitué de N-phénylpyridazone I selon la revendication 1.

**8.** Produit herbicide contenant des additifs inertes et un dérivé substitué de N-phénylpyridazone I selon la revendication 1.

**9.** Procédé pour combattre la croissance de végétaux indésirables, caractérisé en ce que l'on traite les végétaux indésirables par une quantité herbicide efficace d'un dérivé substitué de N-phénylpyridazone I selon la revendication 1.